# EUROPEAN PATENT APPLICATION

(11) **EP 3 677 690 A1**
(43) Date of publication of application: **08.07.2020**
(21) Application number: 18851994.6
(22) Date of filing: 20.08.2018
(51) Int. Cl.: C12Q 1/68, G01N 27/00

(54) **METHOD AND DEVICE FOR ANALYSING NUCLEIC ACIDS**

(30) Priority: 31.08.2017 ES 201700707
(71) Applicant: Medina Venegas, Pedro Manuel, 41920 San Juan de Aznalfarache (Sevilla) (ES); Calvo Villalón, Ignacio, 41920 San Juan de Aznalfarache (Sevilla) (ES)
(72) Inventor: Medina Venegas, Pedro Manuel, 41920 San Juan de Aznalfarache (Sevilla) (ES); Calvo Villalón, Ignacio, 41920 San Juan de Aznalfarache (Sevilla) (ES)
(74) Representative: Rebate Conde, Maria Fernanda
(86) International application number: PCT/ES2018/070564
(87) International publication number: WO 2019/043277

(57) **Abstract**

The invention is made up of a set of systems for receiving and processing samples, for capturing, concentrating and purifying target molecules marked by applying a magnetic field, for energising the marking particles so as to generate a light signal and for acquiring and digitally processing the recorded signal to convert it into a qualitative variable that indicates the presence or absence of the target molecule in a problem sample by means of a test lamp, a screen or any other digital interface or by means of any other system that makes it possible to view the results obtained. Likewise, it is possible to conduct a quantitative analysis of the concentration of the target molecule in the analysed sample by comparing the intensity of the recorded signal with previously calibrated reference values.

## Description

### OBJECT OF THE INVENTION

The present invention relates to a device and a method to be carried out with said device that make it possible to analyse nucleic acids present in all types of samples.

Nucleic acid electrophoresis is a routine procedure in clinics, hospitals and analytical, forensic and research laboratories today. This separation technique is carried out after amplifying the genetic material and is the most widely used method for analysing nucleic acids. The assay is based on the differential migration of said genetic material through an agarose or polyacrylamide polymer gel when an electric field is applied, which gives rise to different bands according to the size of the analysed fragments.

This analysis makes it possible to identify the sequences of interest after staining the generated bands, as well as to recover the desired fragments for subsequent tests and verifications, although with a rather low performance. In any case, electrophoresis is a quite versatile technique that can be adapted to the analytical needs of the user, although it does have certain limitations:
- The bands obtained after separating the fragments can be a result of the overlapping of different sequences, can be non-specific bands of the same size, or they may present a high degree of diffusion, which will prevent correct recognition.
- In some cases, complementary tests may be needed, such as, for example, restriction map analysis, with the consequent increase in costs and time for obtaining conclusive results.
- Every so often, the buffer used in electrophoresis loses its pH regulating capacity and may alter the charge of the sample, compromising the analytical result.
- The use of a molecular weight marker is necessary to determine the size of the band to be analysed. Furthermore, the type of polymer and its concentration in the gel, as well as the migration time, must be adjusted to achieve correct identification of the target sequence, which may generate problems relating to the inability to separate certain similar-sized sequence combinations or to the depolymerisation of the gel due to high exposure times to current.
- Certain types of analysis by gel electrophoresis use polyacrylamide, which is neurotoxic for humans, whereas in the case of agarose, ethidium bromide, a potentially carcinogenic substance, is normally used as a clarifier of the sample. These aspects are usually limiting in laboratories with a high sensitivity to harmful agents and with space limitations, since the use of ethidium bromide, for example, requires having an area that is demarcated and specifically conditioned in order to avoid contamination from other areas of the laboratory.

Furthermore, there is another common method for the recognition of products amplified by PCR or other amplification techniques carried out by laboratories specialising in services of this type: sequencing. This analytical process encompasses a set of quite varied techniques that have evolved considerably in the last few years, although for processing amplification products conventional techniques such as Sanger sequencing are still used. In any case, the use of services of sequencing may have certain drawbacks for users:
- There is a time limit that does not allow immediate results to be obtained, since the service must be outsourced to specialised laboratories which take about 5-7 days to perform the analysis.
- A series of methods and prior controls must be performed in the sample for correct processing thereof without interferences from by-products. Furthermore, a minimum amount of 20 ng of DNA is required for every 100 bases of length of the sequence to be analysed, always maintaining a minimum concentration of 10 ng/µL. To estimate these variables, nucleic acid quantification instruments such as the NanoDrop™ spectrophotometer are commonly used, and it has a series of significant limitations: it does not allow verifying the integrity of the sample as it is unable to distinguish degraded nucleic acids, has a low analytical sensitivity, offers results which may be affected by interferences due to the simultaneous presence of DNA and RNA, and furthermore, its sale price is very high.
- Generally, the length of the sequence to be analysed must be indicated, and in the case of the necessary use of specific primers for sequencing, these must be provided by the client in specific concentration and solvent conditions.

Due to the needs and demands mentioned above, the failed sequencing of fragments of interest is fairly common and affects the continuity of the daily work in laboratories, causing added costs that must be taken on.

Moreover, the main advantages of the proposed invention are the following:
- The proposed device can be used as screening prior to the electrophoresis of fragments amplified by PCR or other amplification techniques, and furthermore, it can replace the electrophoresis in all types of nucleic acid analysis.
- The use of the proposed device can prevent failed results during the identification of the target fragment by means of sequencing due to problems with the specific primers or the amount of DNA, since the developed technology can be used as a test prior to sequencing so as to obtain information that may subsequently be used for the satisfactory identification of the target sequence. Furthermore, it is possible to use the proposed device as an alternative to nucleic acid sequencing as a result of the reliability of the specific sequence analysis that can be performed with same.
- The proposed invention makes it possible to carry out direct analysis of the target molecule with strong enough sensors, without the need for any pre-treatment of the samples, such as, for example, purification or amplification of the target molecule.
- There are no potentially carcinogenic compounds involved in the use of the proposed device, unlike certain substances used in electrophoresis, such as ethidium bromide or acrylamide.
- The analysis time is drastically reduced, going from up to several hours in the case of electrophoresis and prior amplification to just a few minutes with the proposed device.
- The analysis costs are lower with the use of the proposed device, since electrophoresis uses a number of elements that are non-reusable, have a short service life or a high price. Therefore, the proposed device drastically reduces the costs associated with a PCR test with electrophoresis and subsequent sequencing simply by preventing reading by means of electrophoresis, although if this cost reduction is added to the corresponding cost reduction by preventing the sequencing and/or prior amplification of the target molecule, the economic savings are quite substantial.
- In sequence-specific nucleic acid analysis, the proposed device is more reliable than electrophoresis, since multiple identification is performed by sequence, not only by size. Therefore, the main limitation of electrophoresis is overcome and a much more reliable and precise analysis prior to sequencing can be carried out, and it is even possible to dispense with this confirmation test.
- The service life of the proposed device is equivalent to that of the electrophoresis and sequencing equipment, whereas the fungible elements have an established number of tests that can be performed, and the viability thereof does not have to be estimated or checked, as occurs with certain components of analysis by electrophoresis or sequencing.
- The reading of the results obtained after sequencing requires specific programs that may be difficult for non-specialised personnel to use. However, with the developed device a simple result that can be readily interpreted by all types of technical personnel is obtained.
- The proposed device makes it possible reduce highly skilled personnel needs, savings due to the sample processing simplicity, as it is a faster and simpler method that in turn contributes to eliminating the risk of losing the samples when loading the channels with the electrophoresis gels, the failures occurring during the transport of said samples for sequencing or the errors taking place during this process.
- The proposed device represents an affordable alternative for the diagnosis of infectious or genetic diseases, lowering costs for the end client without this entailing a lower performance for users of the system.
- The proposed invention makes it possible carry out a complete analysis without the need for additional or complementary technology, so as no type of additional equipment is required, it makes it possible to perform analysis *in situ* or in the field, and not only in specialised facilities, reducing both the time needed to obtain results and the costs derived from the transport and storage of the samples.
- The proposed device makes it possible to automate the analytical process, whereby considerably increasing the number of samples that can be analysed per unit of time, and therefore laboratory work is optimised and costs per test are reduced.
- The proposed device makes it possible to perform both qualitative analyses which indicate the presence or absence of the target molecule in problem samples and the quantitative analysis of said target molecule for the purpose of determining its concentration in problem samples by digitally processing the recorded signal and comparing it with previously calibrated reference values.
- By using the proposed device and the method for analysis, not only can nucleic acids be analysed, but so can biological macromolecules of another type, particularly proteins, for which it is merely necessary to adapt the probes with which the capture particles are functionalised and the marking of the target molecule.
- In the case of protein analysis, the proposed invention also makes it possible to considerably reduce analysis costs and time corresponding to the use of standard techniques such as protein electrophoresis or Western blot.

The industrial application of this invention falls within the methods for analysis used, among others, in the biotechnology and health sectors, as well as in research in biological sciences.

### BACKGROUND OF THE INVENTION

Although no invention has been found that is identical to the described invention, the documents found that reflect the state of the art related to same are discussed below.

Thus, document ES2607753T3 relates to a method of diagnosing the susceptibility of an individual suffering from a disease to treatment with an HDAC inhibitor, the method comprising assessing the level of expression or activity of a gene or its expression products, or the sequence of a gene, in a tissue sample from a patient and comparing said level of expression or activity or sequence to a reference, wherein a level of expression or activity or sequence that is different from said reference is indicative of an altered susceptibility to treatment with the HDAC inhibitor relative to the reference state, and wherein said gene is Myd88 (myeloid differentiation primary response gene 88). In any case, the mentioned invention does not describe a method or device similar to those proposed for marking the target molecule and obtaining analytical results.

EP0447154A3 describes a device for performing a heterogeneous assay, consisting of: a porous member comprising a plurality of pores to physically entrap, in said porous member, at least one target ligand of a fluid sample, said porous member being adapted for receiving a marked reagent so as to detect the presence or amount of at least said target ligand; and a non-absorbent member having a textured surface, said non-absorbent member being in fluid communication with said porous member, said non-absorbent member being directly in contact and forming a network of capillary channels with said porous member, wherein said fluid communication in said capillary channels is from said porous member towards said non-absorbent member. In any case, the mentioned invention shares neither the method nor the device described by the proposed invention for analysing nucleic acids.

WO2012013731A1 relates to a method for simultaneously detecting and quantifying a microbial nucleic acid in a biological sample, said method comprising: a) isolating and purifying said microbial nucleic acid, b) providing a reaction mixture comprising a first and a second control nucleic acid in different concentrations wherein the first control nucleic acid is a quantitative standard nucleic acid present in a concentration of 20 to 5,000 times the limit of detection of said microbial nucleic acid, and the second control nucleic acid is a qualitative internal control nucleic acid present in a concentration of 1 to 10 times the limit of detection of said microbial nucleic acid, one or more primer pairs specifically hybridising to distinct sequence portions of said microbial nucleic acid and to distinct sequence portions of said control nucleic acids, and probes specifically hybridising to each of the sequences amplified by said one or more primer pairs, wherein said microbial nucleic acid and said first control nucleic acid and said second control nucleic acid hybridise to different probes carrying different markers, c) adding the isolated and purified microbial nucleic acid to said reaction mixture, d) performing one or more cycling steps, wherein a cycling step comprises an amplifying step, said amplifying step comprising producing one or more amplification products derived from said microbial nucleic acid if present in said sample and producing an amplification product derived from said first control nucleic acid and said second control nucleic acid, and wherein a cycling step comprises a hybridising step, said hybridising step comprising hybridising the sequences amplified by said primer pair with said probes, wherein the probes are marked with a donor fluorescent moiety and a corresponding acceptor fluorescent moiety and each of the probes carries a different fluorescent dye, e) detecting and measuring fluorescent signals generated by the amplification products of said first control nucleic acid and said microbial nucleic acid and being proportional to their concentration, and simultaneously detecting fluorescent signals generated by said amplification product of said second control nucleic acid, wherein the presence of an amplification product of said second control nucleic acid is indicative of an amplification occurring in the reaction mixture even in the absence of an amplification product for said microbial nucleic acid, and determining the quantity of said microbial nucleic acid in said biological sample by comparison of the signals generated by said microbial nucleic acid and said first control nucleic acid. In any case, the described method is unrelated to that formulated in the proposed invention for analysing nucleic acids and makes no reference to a device for analysis similar to the once considered in the present invention.

ES2603380A1 describes a piece of equipment and method for detecting protozoa, comprising in a single assembly a sampling device and a protozoa detection kit preferably housed in a portable enclosing box, the sampling device having two variants, one of which is more complex and takes samples over a longer period of time, on the order of days or weeks, while the other, simpler, is indicated for taking samples over a short period of time, on the order of hours at most. The protozoa detection kit includes reagent test strips together with the portable devices and reagents to allow DNA (deoxyribonucleic acid) extraction, PCR (polymerase chain reaction) amplification and hybridisation/development processes to be carried out *in situ.* These reagent strips present the result of the analysis by means of specific markers for the amplified products. In any case, the method considered herein differs from that described in the main invention, and furthermore, there is no mention of device for analysis with the features of the proposed invention.

CN 2010 101935704 B shows a nucleic acid detection system based on two double hybridisations carried out in two phases: in the first hybridisation, magnetic microspheres and gold nanoparticles functionalised with a probe having a specific sequence which will only be retained when applying a magnetic field in the presence of the target DNA are used, and subsequently, said sequence (barcode) is marked with functionalised quantum dots. In any case, there is no mention of the features of a method or a device for analysis similar to those proposed in the present invention, but rather those of a reaction chamber where nucleic acids are amplified and detected are mentioned.

EP 2011 2270202 A1 discloses a detection kit for detecting *Mycobacterium* based on a system of double hybridisation of the target molecule with a capture probe bound to a magnetic microsphere (by means of a streptavidin-biotin bond) and a marking probe bound to a quantum dot. There are provided tables with the specific sequences used and a minimum homology of 90% is required. In any case, the aforementioned invention makes no mention of a device for analysis similar to the one proposed, but rather only mentions the reagents included in the detection kit.

EP 2012 1502101 B1 describes a method for detecting cells and viruses in all types of samples, which may or may not be pre-treated, using marking probes consisting both of oligonucleotides and antibodies conjugated with any signalling molecule, including quantum dots. In parallel, the marked cells are isolated by centrifugation or by applying a magnetic field after being captured, among other proposed methods. However, the aforementioned invention makes no mention of a device for analysis with the features of the proposed invention.

EP 2012 1747295 B1 discloses a general method for detecting analytes consisting of their capture with specific protein or nucleotide probes conjugated with magnetic microspheres and of their marking with nanoparticles functionalised with binding probes for binding to the target molecule and sequences of DNA barcode marked with signalling structures. After double hybridisation, the DNA barcodes are released from the structure by thermal or chemical treatment and finally analysed through the signalling group to determine the presence or absence of the target molecule. In any case, the document does not describe a device similar to the proposed invention for the processing or analysis of the generated signal, nor does it detail the features that the equipment needed for performing the assay must have.

EP 2014 2616557 B1 proposes a system for detecting nucleic acids with double hybridisation with the capture and marking of the target molecule in which a nuclease captured by one of the probes bound to said target molecule intervenes. In this case, the positive result is obtained by reducing the nuclease activity in the reaction mixture with respect to the negative control without the target molecule, or by generating nuclease activity after the release of the enzymes retained in the presence of the target molecule. In any case, a detection kit with the reagents needed for carrying out the assay is patented, but there is no mention of a device for analysis similar to the proposed invention.

US 2002 0028457 A1 is based on a detection method comprising the marking of the target molecule with complementary probes functionalised with quantum dots, optionally amplifying said target molecule for coupling thereto certain chemical groups, for example, biotin, which make the immobilisation thereof on a solid support possible. However, no reference is made to an integrated device for analysis similar to the proposed invention, nor does it contemplate the use of magnetic capture particles.

US 2007 0259359 A1 consists of a method for detecting genetic sequences captured on a solid support based on the marking of the target molecule with probes functionalised with signalling groups, such that the configuration of the probe only makes the degradation thereof and the release of the signalling group possible once it is bound to the target molecule. The detection is carried out using structures made up of electrodes coupled to a substrate in which there are arranged probes complementary to the reporters released by the action of a nuclease, whereby generating a signal due to the variations in recorded electric potential. Furthermore, there are presented methods for the amplification of the signal and array-type structures for carrying out the assay, but there are no references to magnetic capture systems or to a device for analysis with the features of the proposed invention.

US 2007 0269852 A1 contemplates a device for detecting airborne pathogens by means of a system of marking with quantum dot bioconjugates. Said device makes it possible to injection, filter and centrifuge the sample, as well as to obtain a fluorescent signal, although magnetic capture systems are not used, nor does it describe the system for generation and detecting said fluorescent signal, nor does the mentioned device have the features of the proposed invention.

US 2009 0156428 A1 describes a method for the detection based on an array-type structure with molecular probes of all types arranged in any configuration. The document mentions different techniques for marking the probes that are complementary to the target molecule and possible configurations of the assay device that may incorporate this structure of analysis, but the features thereof are not detailed and there is no mention of magnetic capture systems for the target molecule.

US 2010 0086993 A1 consists of a cell detection system based on their capture with ligands for binding to receptors conjugated to magnetic microspheres and their marking with ligands for binding to receptors conjugated with quantum dots. The document also mentions the instruments that could be used for detecting the fluorescent signal generated and the features of the reagents used (composition of the quantum dots, reaction pH, etc.) are defined in a comprehensive manner, but it does not describe a device for analysis with the features of the proposed invention.

US 2010 7799554 B2 proposes a test for detecting nucleic acids based on the use of a lateral flow device having a reaction area and a visualisation area. Furthermore, there are marking probes with a signalling group that is activated only in the presence of the analyte to be detected and capture probes which retain the analyte in the visualisation area. However, there is no mention of a device for analysis with the features of the proposed invention nor are the details concerning its operation described.

US 2011 0152111 A1 contemplates a kit for the simultaneous detection of multiple target sequences based on a system for the selective amplification of the target molecules present in samples of a different type. In this case, there are marking probes functionalised with different chromophores, but the document does not contemplate a device for analysis similar to the proposed invention nor does it mention systems for the capture and concentration of the target molecules.

US 2011 0160090 A1 consists of a lateral flow microarray for detecting single-stranded nucleic acids having a microporous membrane through which a fluid sample moves by capillarity. Furthermore, there is a marking area where a complementary probe functionalised with quantum dots binds to the target molecule and a capture area with immobilised probes. However, magnetic capture particles are not used throughout the process nor is there mention of a device for analysis with the features of the proposed invention.

US 2011 0171749 A1 discloses a system for detecting nucleic acids consisting of the use of marking nanoparticles functionalised with probes specific for each target molecule (single-stranded pathogenic DNA) and magnetic particles functionalised with capture probes. In this case, the sequences of the different probes and the size and features of the nanoparticles used are detailed. Furthermore, different systems for processing the generated signal, which are based on measuring the concentration of the marking particles through electrodes once the excess free nanoparticles are washed, are mentioned. However, systems for generating and detecting light signals are not used nor are the features of a device for analysis similar to the proposed invention detailed.

US 2011 7955802 B2 describes a method for the amplification and detection of nucleic acids using primers functionalised with marking agents and capture probes bound to magnetic spheres attracted by an electromagnet adjacent to the reaction chamber. Amplicons functionalised with said marking agent which are concentrated by the action of a magnetic field are thereby generated. However, the features of the system for energising the marking agent or of the system for the detection and processing of the generated signal are not described. Furthermore, the detection process is not carried out without a prior step of amplification nor is there mention of a device for analysis with the features of the proposed invention.

US 2012 0071330 A1 proposes a method for detecting nucleic acids based on a double hybridisation with the capture and marking of the target molecule, such that the capture probes are immobilised on a solid support. Furthermore, the document contemplates the use of nested probes for amplification of the generated signal and describes a series of methods that make it possible to maximise the proportion of target molecules. However, there is no mention of mechanisms for concentrating said target molecules by means of applying magnetic fields, nor are the mechanisms for detecting the generated signal by the signalling groups of the marking probes detailed, nor is there mention of an integrated device for analysis similar to the proposed invention.

US 2012 8298765 B2 proposes a system for detecting nucleic acids based on the hybridisation of the target molecule with nucleotide monomers capable of polymerising to one another upon binding to said target molecule. In this case, the monomers are marked with quantum dots emitting fluorescence and some features of the energising source responsible for polymerisation and generation of the fluorescent signal are detailed, but there is no mention of mechanisms for capturing the target molecules based on applying magnetic fields, nor is there mention of a device with the features of the proposed invention.

US 2013 0023433 A1 contemplates a system for the identification of nucleic acids based on complex hybridisations that involve their capture and marking. Furthermore, the possibility of incorporating molecular amplifiers in the system and detecting SNP-type mutations is mentioned, but the nature of the capture and marking probes used is not described, nor is there mention of a device with the features of the proposed invention.

US 2013 0085078 A1 consists of a system for detecting nucleic acids by marking with probes immobilised on a solid support provided with a signalling group and hybridisation with a primer that makes it possible for polymerase with exonuclease activity to release the signalling group of the probe bound to the target molecule. In this case, the positive result may be due to an increase or a decrease of the signal by comparison with the initial signal or with unhybridised reference probes. Furthermore, the possibility of amplifying the target molecule before detecting the generated signal is mentioned, but the features of the system for generating, processing and detecting the signal are not detailed, nor are mechanisms for capture by applying magnetic fields mentioned, nor is a device for analysis with the features of the proposed invention described.

US 2013 0123145 A1 proposes an assay kit applicable to various analytical techniques based on quantum dots with different features bound to specific probes of a different nature directed against specific cell markers. However, a device for analysis capable of generating and processing a detectable signal with the features of the proposed invention is not described, nor are mechanisms for capture based on applying magnetic fields mentioned.

US 2013 0172211 A1 consists of a method for detecting genetic sequences of interest based on the generation and subsequent amplification and sequencing of a ligation product resulting from the binding of the different complementary fragments to the target molecule, hybridised consecutively and sequentially in the presence of same. Furthermore, it is mentioned that the process of amplification and detection may involve marking with fluorophores, but mechanisms for capturing the target molecule or the features of a device for analysis similar to the proposed invention are not detailed.

US 2013 8609337 B2 consists of a method for preparing signalling particles wherein a hydrogel microsphere is functionalised with a set of anchor and signalling probes that make it possible to identify and detect specific molecules. In this case, a marking system based on quantum dots is proposed, but there is no mention of mechanisms for molecular capture or of the features of a device for analysis similar to the proposed invention.

US 2014 0024024 A1 contemplates a system for detection capable of sequentially identifying RNA, proteins and DNA in cell and tissue samples, as well as obtaining images of each of the analytical results generated throughout the process. In this case, a process of amplification of the signal prior to the detection thereof is contemplated, such that the intensity of said signal is positively correlated with the concentration of the target molecule present in the sample. Furthermore, the possible fluorescent nature of the signal is mentioned, but mechanisms for molecular capture are not detailed, nor is a device for analysis capable of generating and detecting the signal with the features of the proposed invention described.

US 2014 0332407 A1 consists of a detection kit based on a sensor made up of a pair of electrodes between which there is arranged a structure capable of immobilising a series of receptor molecules retaining target molecules of a different nature upon passage thereof. Subsequently, said target molecules are marked with signalling molecules with conductive properties or capable of placing metal ions in the area located between the electrodes. In any case, although it is mentioned that the detected signal can be both optical and electrical, mechanisms for molecular capture based on applying magnetic fields are not detailed, nor is a device for analysis capable of generating and detecting the signal with the features of the proposed invention described.

US 2014 8691500 B2 contemplates a chamber system where the processing and subsequent analysis of biological samples is performed so as to detect specific analytes. In this case, the target molecule is captured with a magnetic particle and provided with a label, of any origin, which would make it possible to subsequently detect it. However, there is no mention of a module for generating and detecting signal in the system, nor is an integrated device for analysis with the features of the proposed invention described.

US 2015 0004598 A1 proposes different methods for identifying or quantifying analytes in various samples using a nucleic acid as a linker between the analyte to be identified and the marking particle, whether it is a fluorophore, a magnetic sphere, a quantum dot, an antibody, etc. However, there is no mention of a device similar to the proposed invention, since the mentioned patent has as an objective the integration of the method into techniques such as immunohistochemistry, Western blot, hybridisation *in situ*, etc.

US 2016 0231324 A1 proposes an instrument for detecting molecular markers by means of a microfluidic system wherein the sample is embedded in microdroplets and wherein a marking system based on antibodies, enzymes, aptamers and fluorescence sensors is used. In any case, this document relates to an apparatus for detection similar to a flow cytometer, which is devoid of systems for processing the samples and does not present the features of the proposed invention.

US 2016 0265036 A1 proposes a method for identifying and quantifying specific nucleic acids in biological samples by means of double hybridisation with complementary chains: one of them being fixed to a surface and the other one to a phosphate group which, bound to a catalytic enzyme, generates a detectable reaction product. However, an integrated functional device for analysis similar to the proposed invention is not described.

US 2016 0298179 A1 contemplates a microchip for detecting target molecules by means of the binding of signalling groups to luminophores. It is a microarray-type structure, although it uses a different system for marking the target molecule. In fact, it is intended for using the instruments of the analysis with microarrays for carrying out the described assay, and therefore there is no mention of any device whatsoever to be patented as occurs in the case of the proposed invention.

US 2016 9274077 B2 has as an object a system for detecting interactions and bonds between applicable molecules, in addition to polynucleotide sequencing. Said system is based on the use of electrodes and/or an electromagnetic force, but it does not share the features of the integrated device for analysis object of the present invention.

US 2016 9482662 B2 describes a method for detecting biomolecules that involves marking same with different probes and the use of a system of wells adapted for the visualisation of said biomolecules under a microscope, whether it is by using white light or generating fluorescence. However, there is no mention of an integrated functional device for analysis similar to the proposed invention, nor is reference made to processing the analysed samples.

US 2017 9536041 B2 contemplates a method for sequencing single molecules of DNA based on a fluorescent, chemical, electrical or electromagnetic signal and in a multichannel system that also makes it possible to detect the epigenetic pattern. However, a device for generating and acquiring the signal or for processing the samples with the features of the proposed invention is not described.

WO 1998 033939 A1 discloses a system for sequencing single molecules of DNA using magnetic microspheres for fixing the target molecules to a support. Subsequently, once said target molecules are captured, modified nucleotides are incorporated by the action of a polymerase, which nucleotides can be identified as a result of the reflection of ultraviolet radiation made to strike the sample. However, a device for analysis similar to the proposed invention for carrying out this method is not described.

WO 2003 025540 A2 describes a method for analysing nucleic acids using up to three molecular labels, whether they are luminescent or fluorescent molecules, enzymes, radioisotopes, biotins, avidins, semiconductors, colloidal gold, quantum dots, antibodies, haptens, lipids, etc. With this, the signal is amplified so as to increase the resolution of the analysis and achieve a more precise identification of the target molecule, but the mentioned patent does not describe an integrated functional device for analysis similar to the proposed invention.

WO 2005 107818 A2 proposes a method for performing imaging *in vivo* using quantum dots conjugated with molecular labels, whether they are nucleic acids, antibodies, antigens or lipids, in live samples such as tissues or cells of a different type. However, a device for analysis with the features of the proposed invention for carrying out the assay is not described.

WO 2009 012343 A2 proposes an array for detecting a target molecule in a sample; namely, a system for reading that makes it possible to perform the analysis in a microfluidic environment using an injection mechanism. However, a device for analysis with the features of the proposed invention for carrying out the assay is not described.

WO 2010 057264 A1 contemplates a method for the rapid detection of analytes, whether they are nucleic acids, proteins, lipids, antibodies, viruses, bacteria or cells, in aqueous samples using fluorophores or quantum dots. In certain types of analysis, the mentioned system requires a process for digesting double-stranded DNA to enable detecting the subsequently generated signal. In any case, after attaching the labels to the analyte, the spectrum of the recorded signal is analysed, but a device for generating and detecting the signal or for processing the samples with the features of the proposed invention is not described.

WO 2011 038403 A1 relates to a method for molecular detection based on processes of hybridisation under different conditions, but an integrated device similar to the proposed invention, a system for generating or detecting a signal, the nature of the generated signal or the molecular mechanisms for capturing the target molecule are not described.

WO 2012 016357 A1 relates to an array that makes it possible to analyse the interaction between different molecules, whether they are polypeptides, antibodies, carbohydrates, etc. To that end, a marking particle, which may be either a magnetic microsphere, a fluorochrome or a quantum dot, is conjugated to the first problem molecule, and the second problem molecule is fixed to a substrate of a different origin. Therefore, if interaction occurs between the two molecules, a detectable signal will be generated upon applying a magnetic field, an electric field or electromagnetic radiation, but an integrated device similar to the proposed invention making it possible to carry out this analysis is not described.

WO 2016 005517 A1 describes a method for detecting *Leishmaniasis* by amplifying and marking the target molecule with colloidal gold. In this case, direct marking rather than probe-mediated marking of the amplicons of the target molecule is carried out, but there is no mention of a device for analysis similar to the proposed invention for carrying out the assay.

WO 2016 065192 A1 contemplates a method for detecting nucleic acids based on the release of a marker after digesting double-stranded DNA by the action of nucleases on an immobilised and marked sample. However, a device for analysis similar to the proposed invention for carrying out the assay is not described.

WO 2017 008177 A1 proposes a microarray that detects mutations in specific genes by means of a method that involves the use of magnetic marking particles, quantum dots, fluorescent proteins, molecular dyes, etc. However, there is no mention of an integrated functional device for analysis similar to the proposed invention.

Conclusion: As ensues from the investigation that has been performed, none of documents found solves the contemplated problems like the proposed invention does.

### DESCRIPTION OF THE INVENTION

The device for analysing nucleic acids object of the present invention is made up of an integrated structure having the following modules:
- System for receiving samples comprising a set of structures for coupling the containers which contain said samples, as well as the containers in which said samples are placed to perform the analysis.
- System for processing samples provided with an adjustable thermal source that makes it possible to modulate and control the temperature of said samples for the denaturation of the target molecules and the hybridisation of said target molecules with the marking probes and the capture probes incorporated in the samples to take place.
- System for capturing, concentrating and purifying the target molecules marked by applying a magnetic field. In this case, there is a system capable of generating, modulating and applying a magnetic field on the structures for coupling with the containers which contain the samples. This system makes it possible to capture, concentrate and purify the target molecules bound to the capture and marking probes, and subsequently makes it possible to remove the excess marking probes and to remove the capture probes to prevent interferences while acquiring the light signal. Furthermore, this system has a denaturing agent applied manually or automatically to the contents of the containers where processing of the samples is performed so as to release the marking probes and the capture probes retained after the purification step, which makes it possible to remove said capture probes and subsequently generate the light signal by energising the particles bound to the marking probes.
- System for energising the marking particles so as to generate the light signal. This module is made up of a set of LEDs capable of emitting ultraviolet radiation or any other lighting system capable of emitting ultraviolet radiation or capable of emitting any other type of electromagnetic radiation that can energise the marking particles, making said radiation strike the structures for coupling with the containers where the marking probes released after the capture and purification of the marked target molecules are located. Moreover, this system has a series of anti-reflective materials and structures and optical filters arranged such that they maximally reduce light contamination, and therefore interference on the generated signal.
- System for acquiring and processing the light signal through photosensors, amplification structures, digital processing elements and systems for visualising results. This module is made up of a set of photosensors capable of detecting light from the visible spectrum or any other type of light signal, and the position and orientation thereof with respect to the structures for coupling with the containers in which the released marking probes are located make it possible to record in an optimal manner the signal generated by energising the marking particles. Additionally, structures for amplifying the recorded signal, such as, for example, photomultiplier tubes, voltage amplifiers or optical systems, and digital processing elements converting the recorded signal into a qualitative variable that indicates the presence or absence of the target molecule in the analysed sample by means of a test lamp, a screen, or any other digital interface or by means of any other system that makes it possible to view the results obtained are coupled to the system. Likewise, it is possible to conduct a quantitative analysis of the concentration of the target molecule in the analysed sample by comparing the intensity of the recorded signal with previously calibrated reference values.
- Auxiliary systems:
   - Isolating structures that prevent the heat generated by the adjustable thermal source from affecting the operation of other components of the device, and moreover, keep the photosensors protected so as to assure the stability, reliability and robustness of the measurements taken by said photosensors.
   - Power supply systems, electric resistors, switches and any other auxiliary electrical or electronic component of the device.
   - Portable protective casing that houses the set of modules and systems described above in an integrated and functional manner.

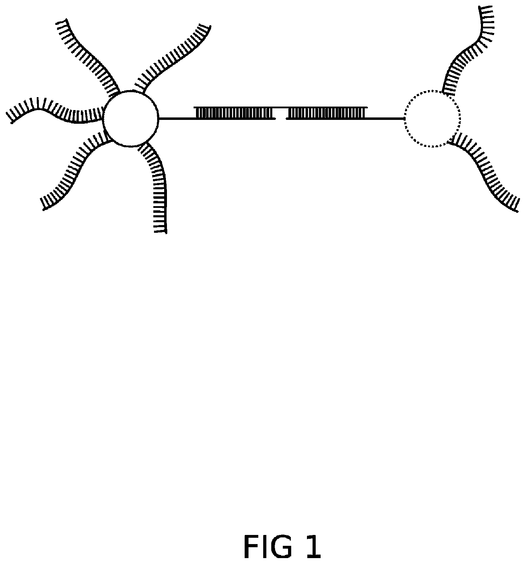

With regard to the analysis carried out using the proposed invention, the assay is based on the molecular principles shown in Figure 1, that make it possible to capture and mark the target molecule.

Moreover, the method for carrying out the nucleic acid analysis making use of the proposed device is developed in the following steps:
i.- Taking as a starting point a problem sample that may contain the target molecule.
ii.- Transferring the problem sample to the initial container and adding the marking particles and the magnetic capture particles functionalised with the corresponding probes.
iii.- Coupling the initial container in the initial receptacle, activating and regulating the thermal source for the problem sample to reach the denaturation temperature, waiting a few minutes once said temperature is reached, adjusting the thermal source for the problem sample to reach the hybridisation temperature and waiting a few minutes once said temperature is reached.
iv.- Deactivating the thermal source, activating the magnetic capture system, waiting a few seconds, removing the supernatant from the initial container, deactivating the magnetic capture system, applying the denaturing agent to the contents of the initial container, gently stirring for a few seconds, activating the magnetic capture system and waiting a few seconds.
v. - Without uncoupling the initial container from the initial receptacle, taking the volume contained in said initial container, which is free of magnetic capture particles, and transferring it to the final container.
vi. - Deactivating the magnetic capture system, activating the system for generating the signal and coupling the final container in the final receptacle so as to see the result obtained by the system for acquiring and processing the generated signal.

In a different embodiment, a system for pumping samples or other systems for injecting samples instead of the device having structures set up for receiving the containers with said samples can be used. In any case, the analysed samples will continue to be subjected to the same treatment; only the manner in which they are made to pass through the device will change.

### DESCRIPTION OF THE DRAWINGS

To better understand the present description, several drawings representing a preferred embodiment of the present invention are attached:
Figure 1: Diagram of the molecular principles on which the nucleic acid analysis carried out using the proposed device is based, with double hybridisation between the target molecule (central horizontal line) and the probe with the capture particle (left) and the probe with the marking particle (right).

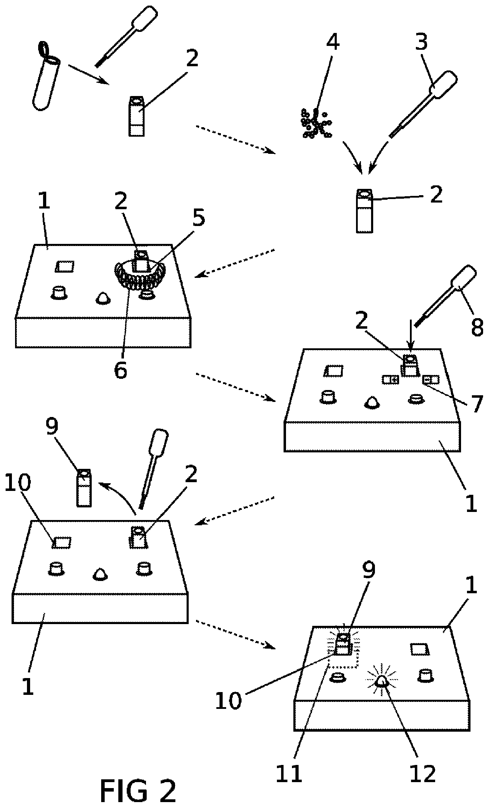

Figure 2: Development of the steps comprising the method for analysing nucleic acids carried out using the device object of the present invention.

The reference numbers appearing in said figures correspond to the following constitutive elements of the invention:
1. Device for analysis
2. Initial container
3. Marking particles
4. Magnetic capture particles
5. Initial receptacle
6. Adjustable thermal source
7. Magnetic capture system
8. Denaturing agent
9. Final container
10. Final receptacle
11. System for generating the signal
12. System for visualising results

### BRIEF DESCRIPTION OF A PREFERRED EMBODIMENT

A preferred embodiment of the device for analysing nucleic acids object of the present invention can be based on an integrated structure having the following modules:
- System for receiving samples comprising a set of structures for coupling the containers which contain said samples, as well as the containers in which said samples are placed to perform the analysis.
- System for processing samples provided with an adjustable thermal source that makes it possible to modulate and control the temperature of said samples for the denaturation of the target molecules and the hybridisation of said target molecules with the marking probes and the capture probes incorporated in the samples to take place.
- System for capturing, concentrating and purifying the target molecules marked by applying a magnetic field. In this case, there is a system capable of generating, modulating and applying a magnetic field on the structures for coupling with the containers which contain the samples. This system makes it possible to capture, concentrate and purify the target molecules bound to the capture and marking probes, and subsequently makes it possible to remove the excess marking probes and to remove the capture probes to prevent interferences while acquiring the light signal. Furthermore, this system has a denaturing agent applied manually or automatically to the contents of the containers where processing of the samples is performed so as to release the marking probes and the capture probes retained after the purification step, which makes it possible to remove said capture probes and subsequently generate the light signal by energising the particles bound to the marking probes.
- System for energising the marking particles so as to generate the light signal. This module is made up of a set of LEDs capable of emitting ultraviolet radiation or any other lighting system capable of emitting ultraviolet radiation or capable of emitting any other type of electromagnetic radiation that can energise the marking particles, making said radiation strike the structures for coupling with the containers where the released marking probes are located after the capture and purification of the marked target molecules. Moreover, this system has a series of anti-reflective materials and structures and optical filters arranged such that they maximally reduce light contamination, and therefore interference on the generated signal.
- System for acquiring and processing the light signal through photosensors, amplification structures, digital processing elements and systems for visualising results. This module is made up of a set of photosensors capable of detecting light from the visible spectrum or any other type of light signal, and the position and orientation thereof with respect to the structures for coupling with the containers in which the released marking probes are located make it possible to record in an optimal manner the signal generated by energising the marking particles. Additionally, structures for amplifying the recorded signal, such as, for example, photomultiplier tubes, voltage amplifiers or optical systems, and digital processing elements converting the recorded signal into a qualitative variable that indicates the presence or absence of the target molecule in the analysed sample by means of a test lamp, a screen, or any other digital interface or by means of any other system that makes it possible to view the results obtained are coupled to the system. Likewise, it is possible to conduct a quantitative analysis of the concentration of the target molecule in the analysed sample by comparing the intensity of the recorded signal with previously calibrated reference values.
- Auxiliary systems:
   - Isolating structures that prevent the heat generated by the adjustable thermal source from affecting the operation of other components of the device, and moreover, keep the photosensors protected so as to assure the stability, reliability and robustness of the measurements taken by said photosensors.
   - Power supply systems, electric resistors, switches and any other auxiliary electrical or electronic component of the device.
   - Portable protective casing that houses the set of modules and systems described above in an integrated and functional manner.

Moreover, the method for carrying out the nucleic acid analysis using the proposed device, with mention of the reference numbers, is developed in the following steps:
i.- Taking as a starting point a problem sample that may contain the target molecule.
ii.- Transferring the problem sample to the initial container (2) and adding the marking particles (3) and the magnetic capture particles (4) functionalised with the corresponding probes.
iii.- Coupling the initial container (2) in the initial receptacle (5), activating and regulating the thermal source (6) for the problem sample to reach the denaturation temperature, waiting a few minutes once said temperature is reached, adjusting the thermal source (6) for the problem sample to reach the hybridisation temperature and waiting a few minutes once said temperature is reached.
iv.- Deactivating the thermal source (6), activating the magnetic capture system (7), waiting a few seconds, removing the supernatant from the initial container (2), deactivating the magnetic capture system (7), applying the denaturing agent (8) to the contents of the initial container (2), gently stirring for a few seconds, activating the magnetic capture system (7) and waiting a few seconds.
v. -Without uncoupling the initial container (2) from the initial receptacle (5), taking the volume contained in said initial container (2), which is free of magnetic capture particles (4), and transferring it to the final container (9).
vi. - Deactivating the magnetic capture system (7), activating the system for generating the signal (11) and coupling the final container (9) in the final receptacle (10) so as to see the result obtained by the system for acquiring and processing the generated signal (12).

## Claims

1. A method for analysing nucleic acids **characterised in that** it comprises the following steps:
i. - Taking as a starting point a problem sample that may contain the target molecule.
ii.- Transferring the problem sample to the initial container (2) and adding the marking particles (3) and the magnetic capture particles (4) functionalised with the corresponding probes.
iii.- Coupling the initial container (2) in the initial receptacle (5), activating and regulating the thermal source (6) for the problem sample to reach the denaturation temperature, waiting a few minutes once said temperature is reached, adjusting the thermal source (6) for the problem sample to reach the hybridisation temperature and waiting a few minutes once said temperature is reached.
iv. - Deactivating the thermal source (6), activating the magnetic capture system (7), waiting a few seconds, removing the supernatant from the initial container (2), deactivating the magnetic capture system (7), applying the denaturing agent (8) to the contents of the initial container (2), gently stirring for a few seconds, activating the magnetic capture system (7) and waiting a few seconds.
v. -Without uncoupling the initial container (2) from the initial receptacle (5), taking the volume contained in said initial container (2), which is free of magnetic capture particles (4), and transferring it to the final container (9).
vi. - Deactivating the magnetic capture system (7), activating the system for generating the signal (11) and coupling the final container (9) in the final receptacle (10) so as to see the result obtained by the system for acquiring and processing the generated signal (12).

2. The method for analysing nucleic acids according to claim 1, **characterised in that** after removing the supernatant not retained by action of the magnetic capture system, a certain volume of a buffer solution is required to be added before applying the denaturing agent.

3. The method for analysing nucleic acids according to claims 1 and 2, **characterised in that** the performance of the analysis does not require transferring the supernatant from one container to another, with it being possible to carry out the assay in a single compartment as the different modules and systems comprised in the device used in the performance of the analysis are integrated therein, such that the processing of the sample and the generation, acquisition and processing of the signal are carried out in an optimal manner and without interferences of any type.

4. The method for analysing nucleic acids according to claims 1 to 3, **characterised in that** upon analysing samples that may contain nucleic acids, it will be possible to establish the negative and positive controls needed to assure the reliability, robustness, sensitivity, specificity, repeatability and reproducibility of the assay.

5. The method for analysing nucleic acids according to claims 1 to 4, **characterised in that** the simultaneous analysis of a plurality of samples can be performed by using a device which has a plurality of systems for receiving samples with structures set up to that effect and comprises a plurality of systems for processing samples and a plurality of systems for generating, acquiring and processing the signal, or at least comprises a plurality of some of the structures forming them.

6. The method for analysing nucleic acids according to claims 1 to 5, **characterised in that** the analysis of all types of problem samples can be performed, regardless of whether or not they have undergone any type of prior treatment such as, for example, the purification or amplification of the target molecule.

7. The method for analysing nucleic acids according to claims 1 to 6, **characterised in that** said method makes it possible to perform the analysis of the target molecule regardless of the nature, size and sequence of said target molecule and whether the sequence of said target molecule is known or not.

8. The method for analysing nucleic acids according to claims 1 to 7, **characterised in that** said method makes it possible to perform the analysis of the target molecule regardless of the nature of the probes, the marking particles and the capture particles used in performing said analysis.

9. The method for analysing nucleic acids according to claims 1 to 8, **characterised in that** said method can be applied to protein analysis by simply adapting the probes with which the capture and marking particles of the target molecule are functionalised.

10. A device for analysing nucleic acids, **characterised in that** it is part of the execution of the method described in the preceding claims and comprises the following systems:
- System for receiving samples comprising a set of structures for coupling the containers which contain said samples, as well as the containers in which said samples are placed to perform the analysis.
- System for processing samples provided with an adjustable thermal source that makes it possible to modulate and control the temperature of the samples for the denaturation of the target molecules and the hybridisation of said target molecules with the marking probes and the capture probes incorporated in the samples to take place.
- System for capturing, concentrating and purifying the marked target molecules that makes it possible to generate, modulate and apply a magnetic field on the structures for coupling with the containers which contain the samples for capturing, concentrating and purifying the target molecules bound to the capture and marking probes, and for subsequently removing the excess marking probes and removing the capture probes to prevent interferences while acquiring the light signal.
- System for energising the marking particles so as to generate the light signal comprising a set of LEDs capable of emitting ultraviolet radiation or any other lighting system capable of emitting ultraviolet radiation or capable of emitting any other type of electromagnetic radiation that can energise the marking particles, making said radiation strike the structures for coupling with the containers where the marking probes released after the capture and purification of the marked target molecules are located. Furthermore, this system also comprises a series of anti-reflective materials and structures and optical filters arranged such that they maximally reduce light contamination, and therefore interference on the generated signal.
- System for acquiring the light signal comprising a set of photosensors capable of detecting light from the visible spectrum or any other type of light signal, and the position and orientation thereof with respect to the structures for coupling with the containers in which the released marking probes are located make it possible to record in an optimal manner the signal generated by energising the marking particles.

11. The device for analysing nucleic acids according to claim 10, **characterised in that** structures for amplifying the recorded signal, such as, for example, photomultiplier tubes, voltage amplifiers or optical systems, and digital processing elements converting said recorded signal into a qualitative variable that indicates the presence or absence of the target molecule in the analysed sample by means of a test lamp, a screen, or any other digital interface or by means of any other system that makes it possible to view the results obtained are additionally coupled to the system for acquiring the light signal.

12. The device for analysing nucleic acids according to claims 10 and 11, **characterised in that** makes it possible to conduct a quantitative analysis of the concentration of the target molecule in the analysed sample as a result of the digital processing of the intensity of the recorded signal and its comparison with previously calibrated reference values.

13. The device for analysing nucleic acids according to claims 10 to 12, **characterised in that** it comprises the following auxiliary systems: isolating structures that prevent the heat generated by the adjustable thermal source from affecting the operation of other components of the device, and that, moreover, keep the photosensors protected so as to assure the stability, reliability and robustness of the measurements taken by said photosensors; power supply systems, electric resistors, switches and any other auxiliary electrical or electronic component of the device. Furthermore, said device also comprises a portable protective casing that houses the set of modules and systems described in preceding claims in an integrated and functional manner.

14. The device for analysing nucleic acids according to claims 10 to 13, **characterised in that** the samples are provided by means of a pumping or injection system which causes them to pass through the device to perform the analysis instead of being transferred to containers that can be coupled in structures set up to that effect.

15. The device for analysing nucleic acids according to claims 10 to 14, **characterised in that** said device makes it possible to perform the analysis of the target molecule directly and without the need for additional or complementary technology, which makes it possible to perform the analysis both *in situ* or in the field and in laboratories and in any other type of facilities fitted and equipped for performing molecular analysis.

16. The device for analysing nucleic acids according to claims 10 to 15, **characterised in that** the analytical method carried out with said device can be automated by making use of additional software and hardware elements.

17. The device for analysing nucleic acids according to claims 10 to 16, **characterised in that** said device can be applied to protein analysis by simply adapting the probes with which the capture and marking particles of the target molecule are functionalised.
